Europäisches Patentamt

European Patent Office

Office européen des brevets

(11) Publication number: **0 137 832**
**B1**

(12) # EUROPEAN PATENT SPECIFICATION

(45) Date of publication of patent specification: **12.08.87**

(21) Application number: **84901209.1**

(22) Date of filing: **12.03.84**

(86) International application number:
**PCT/HU84/00016**

(87) International publication number:
**WO 84/03442 13.09.84 Gazette 84/22**

(51) Int. Cl.⁴: **A 61 K 31/53,** A 61 K 31/35,
A 61 K 31/535, A 61 K 31/70,
A 61 K 31/37

(54) PHARMACEUTICAL COMPOSITIONS OF ANTI-PANCREATIC INFLAMMATORY EFFECT.

(30) Priority: **11.03.83 HU 84283**

(43) Date of publication of application:
**24.04.85 Bulletin 85/17**

(45) Publication of the grant of the patent:
**12.08.87 Bulletin 87/33**

(84) Designated Contracting States:
**AT BE CH DE FR GB LI LU NL SE**

(56) References cited:
**EP-A-0 028 660
EP-A-0 034 270
DE-A-3 047 487
DE-B-1 470 296
DE-B-2 206 570
FR-A-2 437 838
FR-M- 917
FR-M- 1 611
GB-A-1 589 294
GB-A-1 595 351**

(73) Proprietor: **BIOGAL GYOGYSZERGYAR
Pallagi u. 13.
H-4042 Debrecen (HU)**

(73) Proprietor: **ALKALOIDA VEGYéSZETI GYáR
Postfach 1
H-4440 Tiszavasvári (HU)**

(72) Inventor: **MADI-SZABO, László
20, Mátyáshegyi ut
H-1037 Budapest (HU)**
Inventor: **MORVAI, Margit
20, Mátyáshegyi ut
H-1037 Budapest (HU)**
Inventor: **HORVATH, Eva née FEHER
33, Naphegy u.
H-1016 Budapest (HU)**
Inventor: **JANCSO, Sándor
28, Kardos u.
H-Debrecen (HU)**
Inventor: **TAMASI, Piroska
24, Bem tér
H-Debrecen (HU)**
Inventor: **KOVACS, István
86, Csapó u.
H-Debrecen (HU)**
Inventor: **KISS, Ilona, Mária née LOOS
12, Monostor u.
H-Debrecen (HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## 0 137 832

(56) References cited:

M. Negwer, "Organisch-Chemische Arzneimittel und ihre Synonyma" Volume I, published 1978, Akademie-Verlag (Berlin) - see page 565, rference 3117, see pages 447-448, rferences 2486, 2487, see page 496,references 2761, 2762. Volume II, published 1978, Akademie-Verlag (Berlin), see page 1040, rference 5573, see page 1113, references 5897, 5898.

P.H. List, L. Hörhammer, "Hagers Handbuch der Pharmazeutischen Praxis", fourth edition, volume 2, published 1969, Springer-Verlag (Berlin, Heidelberg, New York), see pages 714-717.

(72) Inventor: **BACSA, György**
**90, Csapó u.**
**H-Debrecen (HU)**
Inventor: **KUBALA, Mária née PAPP**
**4, Elmunkás u.**
**H-Tiszavasvári (HU)**
Inventor: **BENE, Magdolna née HORVATH**
**23, Elmunkás u.**
**H-Tiszavasvári (HU)**

(74) Representative: **Patentanwälte Viering &**
**Jentschura**
**Steinsdorfstrasse 6**
**D-8000 München 22 (DE)**

**Description**

The present invention relates to synergistic pharmaceutical compositions with anti-pancreatic inflammatory activity and a process for preparing the same.

In the last two decades chronic inflammatory diseases of the pancreas have caused increased clinical problems. This phenomenon is due to, on one hand, the increased number of patients, and on the other hand to the increased number of diagnostic methods. These facts are referred to in the following publication: Marks, J. N., Bank, S., Louw, I. H.: Leber, Magen, Darm. 6 257, 1976.

It is known in the art that the pancreatic inflammatory processes show specific features (Papp, M.: Congress of the Hungarian Gastroent. Society, Keszthely, Hungary, 1981).

The inflammation arising from either ductal or parenchimic features leads to the deterioration of parenchyma, progressive histolysis, fibrotizing processes and cicatrization. The constant presence of free digestive enzymes which maintain autoperpetuation is induced by the inflammatory mechanism, the edematic processes and the trophic disorders. Besides the inflammational edema the compression of the subcapsular limphatics by that also diminishes the microcirculation. G. Kaiser's and G. Hommel's observation is also of great importance. According to their report the decreasing blood supply to the pancreas under deterioration induces adaptive intimafibrosis in the small blood-vessels. This process further reduces the amount of blood amount flowing to the gland and it can be the basis of further athropy. (Kaiser, G., Hommel, G.: Morphometrisch-statistische Analyse der Pankreasarterien bei chronischer Pankreatitis. Virchows Arch (Pathol. Anat.) *365* 103 (1975)).

In spite of the theoretical considerations above there are not yet available pharmaceutical compositions with satisfactory efficiency for the treatment of pancreatic inflammations. In order to relieve the chronic pancreatitis patient's complaints and symptoms the medical treatment is limited to administering enzyme substituents with simultaneous diet. Such enzyme preparations are e.g. Dipankrine, Cotasym Forte or Panpur.

The aim of our research work was to discover a pharmaceutical composition which is suitable for the efficient treatment of patients suffering from pancreatic inflammations and for the cure of pancreatitis. According to our observation steroidal antiinflammatory agents tend to induce recidiva while the prostaglandin antagonists are powerless.

Surprisingly we have found that the pharmaceutical compositions containing 3 - dimethylamino - 7 - methyl - 1,2 - (n - propylmalonyl) - 1,2 - dihydro - 1,2,4 - benzotriazine and at least one compound selected from 3',4',7 - tris(β - hydroxyethyl) - rutin and (+) - 2 - (3,4 - dihydroxyphenyl) - 3,5,7 - chromantriol as active ingredients are effective against pancreatic inflammations.

The weight ratio of 3 - dimethylamino - 7 - methyl - 1,2 - (n - propylmalonyl) - 1,2 - dihydro - 1,2,4 - benzotriazine and 3',4',7 - tris(β - hydroxyethyl) - rutin is between 2:1 to 1:1 in the compositions of the invention.

The weight ratio of 3 - dimethylamino - 7 - methyl - 1,2 - (n - propylmalonyl) - 1,2 - dihydro - 1,2,4 - benzotriazine and (+) - 2 - (3,4 - dihydroxyphenyl) - 3,5,7 - chromantriol varies within the range of 2:1 to 1:1 in the compositions of the invention.

The compositions according to the invention suitably contain 60 to 300 mg of 3 - dimethylamino - 7 - methyl - 1,2 - (n-propylmalonyl) - 1,2 - dihydro - 1,2,4 - benzotriazine and a total of 60 to 300 mg of 3'4',7 - tris(β - hydroxyethyl) - rutin and/or (+) - 2 - (3,4 - dihydroxyphenyl) - 3,5,7 - chromantriol per dosage unit.

The pharmaceutical compositions according to the invention optionally contain one or more known antibiotics and/or trace elements and/or vitamins in association with carrier(s), excipient(s) and diluent(s) conventionally used in the preparation of pharmaceutical compositions.

As antibiotics Penicillin V or Tobramycin are preferred.

The compositions of the invention optionally contain vitamins such as e.g. magnesium ascorbate, ascorbic acid or other conventionally used vitamins.

As carriers or diluents those conventionally used, e.g. talc, calcium carbonate, magnesium stearate, starch, dextrin, water physiological saline or polyethylene glycols can be used.

The compositions according to the invention contain as excipients conventional excipients, for example emulsifying agents, disintegrating agents or sweeteners.

Preferred pharmaceutical forms of the present invention are solid forms such as tablets, pills capsules, dragees, granules, or liquid forms, such as solutions, suspensions, emulsion, syrups and injection and infusion solutions.

These compositions are administered orally, by intravenous or subcutane injections or by infusions.

Clinical results obtained by the administration of 3 - dimethylamino - 7 - methyl - 1,2 - (n-propylmalonyl) - 1,2 - dihydro - 1,2,4 - benzotriazine (Azapropazone)

As it is well known from pharmacological and pharmacokinetical experiments, Azapropazone is absorbed well from the duodenum and spreads into the extracellular space. In inflammed tissues it enriches to 3 to 8-fold amount measured in edemic liquids when compared to other ones.

It is not decomposed in the organism, it is not metabolised in liver and it is discharged in the urine. The ulcerogenic effect of the compound is negligible, damage to the liver and medullar is not observed, and it

**0 137 832**

does not possess teratogenic and carcinogenic effects. It does not influence detectably the function of the organs, does not cause an increase in body weight, the composition of the urine and the blood. It has a slight influence on the anabolic function of cells, but does not change either the cell production or the specific synthesis thereof, thus it does not interfere with mucopolysaccharide production and therefore does not interfere with the reparation process. However, it restrains the catabolic anabolic processes by its effect on the mediators of the inflammation. It blocks the course of the inflammatory processes at the level of the deliberation or the function of the lysosomal enzymes ("lysosomal stabilizing effect").

The results of the treatment with Azapropazone as the active ingredients are shown in Table 1.

TABLE 1

| Number of patients | | | Pathography | | | |
|---|---|---|---|---|---|---|
| 20 patients | cholecysto-pancreatitis (with stones) | cholangiopancr. (st.p.chole-cystectomiam) | Chol. pancr.+ diabetes | Panc. chr. alc. | Panc. chr. alc.+ ps.cysts |
| men: 7 | — | 1 | 1 | 4 | 1 |
| women: 13 | 7 | 4 | 1 | 1 | — |
| clinical effect | | | | | |
| insignificant | 5 | 4 | 1 | 2 | 1 |
| medium | 2 | 1 | — | 3 | — |
| excellent | — | — | — | — | — |

Daily 3×300 mg of Azapropazone were administered orally.
The treatment was carried out for 12 weeks.

Clinical results obtained by the administration of hydroxyrutin or catechin alone and combinations according to the invention

In order to increase the antiinflammatory effect, compounds with vascular activity were simultaneously tested.

The compounds are 3′,4′,7 - tris(β - hydroxyethyl)rutin (referred to as Hydroxyrutin) and (+) - 2 - (3,4 - dihydroxyphenyl)-3,5,7 - chromantriol (referred to as Catechin).

Having demonstrated that the acute toxicity of Azapropazone is not increased by the presence of Hydroxyrutin or Catechin, further experiments were carried out.

In the first step of these experiments Hydroxyrutin or Catechin were administered to the patients alone. The results are shown in Tables 2 and 3.

## TABLE 2

| Number of patients | | | Pathography | | | |
|---|---|---|---|---|---|---|
| 20 patients | cholecysto-pancreatitis (with stones) | cholangiopancr. (st.p. chole-cystectomiam) | chol. pancr.+ diabetes | chol. pancr.+ ps.cysta | Panc. chr. alc. | Panc. chr.+ ps.cysts |
| men: 5 | 1 | — | — | — | 3 | 1 |
| women: 15 | 5 | 5 | 2 | 1 | 2 | — |
| clinical effect insignificant 6 | 2 | 2 | — | 1 | — | 1 |

Daily 3×300 mg of Hydroxyrutin were administered orally.
The treatment was carried out for 12 weeks.

## TABLE 3

| Number of patients | | | Pathography | | | |
|---|---|---|---|---|---|---|
| 20 patients | cholecysto-pancreatitis (with stones) | cholangiopancr. (st.p.cholescyst-ectomiam) | chol. pancr.+ diabetes | chol. pancr.+ ps.cysta | Panc. chr. alc. | Panc. chr.+ ps.cysts |
| men: 7 | 2 | 1 | — | — | 3 | 1 |
| women: 13 | 5 | 4 | 1 | 1 | 2 | 1 |
| clinical effect insignificant 6 | 1 | 2 | — | 1 | 1 | 1 |

Daily 3×300 mg of Catechin were administered orally to the patients.
The treatment was carried out for 12 weeks.

As is shown by the Tables, the administration of Hydroxyrutin or Catechin alone resulted in an insignificant effect.

Surprisingly, the simultaneous administration of Azapropazone and Hydroxyrutin or Azapropazone and Catechin resulted in an unexpected effect. The anti-pancreatic inflammatory effect of Azapropazone was highly synergized by the compounds of vascular activity employed in the experiments. The results are shown in Table 4.

# TABLE 4

| Number of patients | Combination of compounds applied | Pathography | | | | | | |
|---|---|---|---|---|---|---|---|---|
| 50 patients | Azapropazone+ hydroxy-rutin | Chole-cysto pancr. (with stones) | Chol. pancr. (st.p. chole-cysteco-miam) | Chol. angio pancr. +diabetes | Chol-angio pancr.+ ps.cysta | Panc. chr. alc. | Pancr. chron. alc.+ diabetes | Pancr. chron. alc.+ ps.cysts |
| men: 16 | | 2 | 2 | 1 | 2 | 2 | 3 | 4 |
| women: 34 | | 10 | 10 | 6 | 2 | 5 | 1 | — |
| clinical effect | | | | | | | | |
| insufficient | — | — | — | — | — | — | — | — |
| medium | 18 | 7 | 3 | 4 | 1 | — | 2 | 1 |
| excellent | 32 | 5 | 9 | 4 | 3 | 7 | 1 | 3 |
| men: 16 | Azapropa-zone+ Catechin | 1 | 2 | 1 | 2 | 1 | 3 | 4 |
| woman: 36 | | 10 | 11 | 6 | 3 | 5 | 1 | — |
| clinical effect | | | | | | | | |
| insufficient | — | — | — | — | — | — | — | — |
| medium | 15 | 6 | 3 | 3 | 1 | 1 | — | 1 |
| excellent | 35 | 6 | 8 | 5 | 3 | 8 | 3 | 2 |

Daily 3×300 mg of Azapropazone and 3×300 mg of Hydroxyrutin or 3×300 mg of Catechin were administered to the patients. The treatment was carried out for 12 weeks in the case of both combinations.

Abbreviations applied in Tables 1 to 4:
cholangiopancr.=cholangiopancreatitis
st.p.cholecystectomiam=status post cholecystectomiam
chol.pancr.+diabetes=cholangipancreatitis+diabetes
panc.chr.alc.=alcoholic chronic pancreatitis
panc.chr.alc.+ps.cysta=alcoholic chronic pancreatitis+pancreas cysts

0 137 832

**0 137 832**

Modes of carrying out the invention
The invention is illustrated by the following, non-limiting examples.

Example 1

Composition of a tablet:

| | |
|---|---|
| Azapropazone | 300 mg. |
| Hydroxyrutin | 300 mg. |
| Carrier | q.s. for a tablet |

Example 2

Composition of a tablet:

| | |
|---|---|
| Azapropazone | 300 mg. |
| Hydroxyrutin | 150 mg |
| Catechin | 150 mg. |
| Carrier | q.s. for a tablet |

Example 3

Composition of a tablet:

| | |
|---|---|
| Azapropazone | 300 mg. |
| Hydroxyrutin | 150 mg. |
| Ascorbic acid | 100 mg. |
| Carrier | q.s. for a tablet |

Example 4

Composition for a tablet:

| | |
|---|---|
| Azapropazone | 150 mg. |
| Catechin | 150 mg. |
| Carrier | q.s. for a tablet |

Example 5

Composition for a dragée:

| | |
|---|---|
| Azapropazone | 150 mg. |
| Hydroxyrutin | 150 mg. |
| Magnesium ascorbate | 150 mg. |
| Penicillin V | 30 mg. |
| Tobramycin | 40 mg. |
| Carrier | q.s. for a dragée |

Example 6

Composition for a capsule:

| | |
|---|---|
| Azapropazone | 150 mg. |
| Hydroxyrutin | 150 mg. |
| Carrier | q.s. for a capsule |

Example 7

Composition for a capsule:

| | |
|---|---|
| Azapropazone | 150 mg. |
| Catechin | 150 mg. |
| Sodium ascorbate | 30 mg. |
| Carrier | q.s. for a capsule |

Example 8

Composition for a drinkable liquid (drops):

| | |
|---|---|
| Azapropazone | 300 mg. |
| Hydroxyrutin | 300 mg. |
| Vitamin C | 3 mg. |
| Diluent | q.s. 100 ml. |

Example 9

Composition for an injectable preparation:

| | |
|---|---|
| Azapropazone | 3 g. |
| Hydroxyrutin | 3 g. |
| Penicillin V | 30 mg. |
| Dissolved in physiological saline, filled into 2.0 to 5.0 ml ampoules after sterile filtration | q.s. 1000 ml. |

8

**Claims for the Contracting States: BE, CH, DE, FR, GB, LI, LU, NL, SE**

1. Pharmaceutical compositions comprising 3 - dimethylamino - 7 - methyl - 1,2 - (n - propylmalonyl) - 1,2 - dihydro - 1,2,4 - benzotriazine and at least one compound selected from 3',4',7 - tris(β - hydroxyethyl)rutin and (+) - 2 - (3,4 - dihydroxyphenyl) - 3,5,7 - chromantriol as active ingredients.

2. Pharmaceutical compositions as claimed in claim 1 comprising one or more antibiotics and/or trace elements and/or vitamins and carrier(s), diluent(s), excipient(s) generally used in the preparation of pharmaceutical compositions.

3. Pharmaceutical compositions as claimed in claim 1 or 2 comprising 60 to 300 mg of 3 - dimethylamino - 7 - methyl - 1,2 - (n - propylmalonyl) - 1,2 - dihydro - 1,2,4 - benzotriazine per dosage unit.

4. Pharmaceutical compositions as claimed in claims 1 to 3 comprising 60 to 300 mg of 3',4',7 - tris(β - hydroxyethyl)rutin and/or (+) - 2 - (3,4 - dihydroxyphenyl) - 3,5,7 - chromantriol per dosage unit.

5. Pharmaceutical compositions as claimed in claims 1 to 3 comprising 3 - dimethylamino - 7 - methyl - 1,2 - (n - propylmalonyl) - 1,2 - dihydro - 1,2,4 - benzotriazine and 3',4',7 - tris(β - hydroxyethyl)rutin in a weight ratio of 2:1 to 1:1.

6. Pharmaceutical compositions as claimed in any of claims 1 to 3 comprising 3 - dimethylamino - 7 - methyl - 1,2 - (n - propylmalonyl) - 1,2 - dihydro - 1,2,4 - benzotriazine and (+) - 2 - (3,4 - dihydroxyphenyl) - 3,5,7 - chromantriol in a weight ratio of 2:1 to 1:1.

7. A process for the preparation of pharmaceutical compositions which comprises admixing 3 - dimethylamino - 7 - methyl - 1,2 - (n - propylmalonyl) - 1,2 - dihydro - 1,2,4 - benzotriazine and at least one compound selected from 3',4',7 - tris(β - hydroxyethyl)rutin and (+) - 2 - (3,4 - dihydroxyphenyl) - 3,5,7 - chromantriol and optionally one or more antibiotics and/or trace elements and/or vitamins with excipient(s), carrier(s), diluent(s) conventionally used in the preparation of pharmaceutical compositions and transforming the obtained mixture into a pharmaceutical composition in a known manner.

**Claims for the Contracting State: AT**

1. A process for the preparation of pharmaceutical compositions which comprises admixing 3 - dimethylamino - 7 - methyl - 1,2 - (n - propylmalonyl) - 1,2 - dihydro - 1,2,4 - benzotriazine and at least one compound selected from 3',4',7 - tris(β - hydroxyethyl)rutin and (+) - 2 - (3,4 - dihydrophenyl) - 3,5,7 - chromantriol and optionally one or more antibiotics and/or trace elements and/or vitamins with excipient(s), carrier(s), diluent(s) conventionally used in preparation of pharmaceutical compositions and transforming the obtained mixture into a pharmaceutical composition in a known manner.

2. A process as claimed in claim 1 which comprises admixing 60 to 300 mg of 3 - dimethylamino - 7 - methyl - 1,2 - (n - propylmalonyl) - 1,2 - dihydro - 1,2,4 - benzotriazine per dosage unit.

3. A process as claimed in any of claims 1 or 2 which comprises admixing 60 to 300 mg of 3',4',7 - tris(β - hydroxyethyl)rutin and/or (+) - 2 - (3,4 - dihydroxyphenyl) - 3,5,7 - chromantriol per dosage unit.

4. A process as claimed in any of claims 1 to 3 which comprises admixing 3 - dimethylamino - 7 - methyl - 1,2 - (n - propylmalonyl) - 1,2 - dihydro - 1,2,4 - benzotriazine and 3',4',7 - tris(β - hydroxyethyl)rutin in a weight ratio of 2:1 to 1:1.

5. A process as claimed in claims 1 to 4 which comprises admixing 3 - dimethylamino - 7 - methyl - 1,2 - (n - propylmalonyl) - 1,2 - dihydro - 1,2,4 - benzotriazine and (+) - 2 - (3,4 - dihydroxyphenyl) - 3,5,7 - chromantriol in a weight ratio of 2:1 to 1:1.

**Patentansprüche für die Vertragsstaaten: BE, CH, DE, FR, GB, LI, LU, NL, SE**

1. Pharmazeutische Verbindung mit 3 - Dimethylamino - 7 - Methyl - 1,2 - (n - Propyl - malonyl) - 1,2 - Dihydro - 1,2,4 - Benzotriazin und wenigstens einer Verbindung von 3',4',7 - Tris(β - Hydroxyäthyl)- Rutin und (+) - 2 - (3,4 - Dihydroxyphenyl) - 3,5,7 - Chromantriol als aktiven Bestandteil.

2. Pharmazeutische Verbindung nach Anspruch 1 mit einem oder mehreren Antibiotika und/oder Spurenelementen und/oder Vitamin und Trägersubstanzen, Streckstoffen und Mitteln, wie sie zur Herstellung pharmazeutischer Verbindungen generell verwendet werden.

3. Pharmazeutische Verbindung nach Anspruch 1 oder 2 mit 60 bis 300 mg. 3 - Dimethylamino - 7 - Methyl - 1,2 - (n - Propylmalonyl) - 1,2 - Dihydro - 1,2,4 - Benzotriazin per Dosiseinheit.

4. Pharmazeutische Verbindung nach den Ansprüchen 1 bis 3 mit 60 bis 300 mg 3',4',7 - Tris(β-Hydroxyäthyl)Rutin und/oder (+) - 2 - (3,4 - Dihydroxyphenyl) - 3,5,7 - Chromatriol per Dosiseinheit.

5. Pharmazeutische Verbindung nach den Ansprüchen 1 bis 3 mit 3 - Dimethylamino - 7 - Methyl - 1,2 - (n - Propylmalonyl) - 1,2 - Dihydro - 1,2,4 - Benzotriazin und 3',4',7 - Tris(β - Hydroxyäthyl)Rutin in einem Gewichtsverhältnis von 2:1 bis 1:1.

6. Pharmazeutische Verbindung nach den Ansprüche 1 bis 3 mit 3 - Dimethylamino - 7 - Methyl - 1,2 - (n - Propylmalonyl) - 1,2 - Dihydro - 1,2,4 - Benzotriazin und (+) - 2 - (3,4 - Dihydroxyphenyl) - 3,5,7 - Chromantriol in einem Gewichtsverhältnis von 2:1 bis 1:1.

7. Verfahren zur Herstellung einer pharmazeutischen Verbindung, bei welchem 3 - Dimethylamino - 7 - Methyl - 1,2 - (*n* - Propylmalonyl - 1,2 - dihydro - 1,2,4 - Benzotriazin und wenigstens eine Verbindung aus 3′,4′,7 - Tris(β - Hydroxyäthyl)Rutin und (+) - 2 - (3,4 - Dihydroxyphenyl) - 3,5,7 - Chromantriol und zusätzlich ein oder mehrere Antibiotika und/oder Spurenelemente und/oder Vitamine mit Mitteln, Trägersubstanzen und Streckstoffen, wie sie gewöhnlich bei der Verarbeitung pharmazeutischer Verbindung Verwendung finden, beigemischt werden und die erhaltene Mischung auf bekannte Weise in einer pharmazeutische Verbindung überführt wird.

**Patentansprüche für Vertragsstaat: AT**

1. Verfahren zur Herstellung einer pharmazeutischen Verbindung, bei welchem 3 - Dimethylamino - 7 - Methyl - 1,2 - (*n* - Propylmalonyl) - 1,2 - Dihydro - 1,2,4 - Benzotriazin und wenigstens eine Verbindung aus 3′,4′,7 - Tris(β - Hydroxyethyl)Rutin und (+) - 2 - (3,4 - Dihydrophenyl) - 3,5,7 - Chromantriol und wahlweise ein oder mehrere Antibiotica und/oder Spurenelemente und/oder Vimamine mit Mitteln, Trägersubstanzen und Streckstoffen, wie sie gewöhnlich bei der Herstellung pharmazeutischer Verbindungen verwendet werden, gemischt werden und die erhaltene Mischung in bekannter Weise in eine pharmazeutische Verbindung überführt wird.

2. Verfahren nach Anspruch 1, bei welchem 60 bis 300 mg. 3 - Dimethylamino - 7 - Methyl - 1,2 - (*n* - propylmalonyl) - 1,2 - Dihydro - 1,2,4 - Benzotriazin per Dosiseinheit beigemischt werden.

3. Verfahren nach Anspruch 1 oder 2, bei welchem 60 bis 300 mg. 3′,4′,7 - Tris(β - Hydroxyäthyl)Rutin und/oder (+) - 2 - (3,4 - Dihydroxyphenyl) - 3,5,7 - Chromantriol per Dosiseinheit beigemischt werden.

4. Verfahren nach einem der Ansprüche 1 bis 3, bei welchem 3 - Dimethylamino - 7 - Methyl - 1,2 - (*n* - Propylmalonyl) - 1,2 - Dihydro - 1,2,4 - Benzotriazin und 3′,4′,7 - Tris(β - Hydroxyäthyl)Rutin in einem Gewichtsverhältnis von 2:1 bis 1:1 beigemischt werden.

5. Verfahren nach einem der Ansprüche 1 bis 4, bei welchem 3 - Dimethylamino - 7 - Methyl - 1,2 - (*n* - Propylmalonyl) - 1,2 - Dihydro - 1,2,4 - Benzotriazin und (+) - 2 - (3,4 - Dihydroxyphenyl) - 3,5,7 - Chromantriol in einem Gewichtsverhältnis von 2:1 bis 1:1 beigemischt werden.

**Revendications pour les Etats Contractants: DE, CH, DE, FR, GB, LI, LU, NL, SE**

1. Compositions pharmaceutiques comprenant de la 3 - diméthylamino - 7 - méthyl - 1,2 - (n - propylmalonyl) - 1,2 - dihydro - 1,2,4 - benzotriazine et au moins l′un d′entre les deux corps suivants: 3′,4′,7 - tris(β - hydroxyéthyl)rutine et (+) - 2 - (3,4 - dihydroxyphényl) - 3,5,7 - chromanetriol, comme ingredients actifs.

2. Compositions pharmaceutiques selon la revendication 1, comprenant un ou plusieurs antibiotiques et/ou des éléments traces et/ou des supports, diluants, excipients généralement utilisés dans la préparation de compositions pharmaceutiques.

3. Conpositions pharmaceutiques selon la revendication 1 ou 2, comprenant de 60 à 300 mg de 3 - diméthylamino - 7 - méthyl - 1,2 - (n-propylmalonyl) - 1,2 - dihydro - 1,2,4 - benzotriazine par unité de dosage.

4. Compositions pharmaceutiques selon les revendications 1 à 3, comprenant de 60 à 300 mg de 3′,4′,7 -tris (β - hydroxyéthyl)rutine et/ou (+) - 2 - (3,4 - dihydroxyphényl) - 3,5,7 - chromanetriol par unité de dosage.

5. Compositions pharmaceutiques selon les revendications 1 à 3, comprenant de la 3 - diméthylamino - 7 - méthyl - 1,2 - (n - propylmalonyl) - 1,2 - dihydro - 1,2,4 - benzotriazine et de la 3′,4′,7 - tris(β - hydroxyéthyl)rutine dans un rapport pondéral de 2/1 à 1/1.

6. Compositions pharmaceutiques selon les revendications 1 à 3, comprenant de la 3 - diméthylamino - 7 - méthyl - 1,2 - (n - propylmalonyl) - 1,2 - dihydro - 1,2,4 - benzotriazine et du (+) - 2 - (3,4 - dihydroxyphényl) - 3,5,7 - chromanetriol dans un rapport pondéral de 2/1 à 1/1.

7. Un procédé de préparation de compositions pharmaceutiques qui consiste à mélanger de la 3 - diméthylamino - 7 - méthyl - 1,2 - (n - propylmalonyl) - 1,2 - dihydro - 1,2,4 - benzotriazine et au moins un composé choisi parmi la 3′,4′,7 - tris(β-hydroxyéthyl)rutine et le (+) - 2 - (3,4 - dihydroxyphényl) - 3,5,7 - chromanetriol et éventuellement un ou plusieurs antibiotiques et/ou un ou plusieurs éléments traces et/ou une ou plusieurs vitamines avec des excipients, supports, diluants utilisés de façon classique dans la préparation des compositions pharmaceutiques et à transformer le mélanger obtenu en une composition pharmaceutique d′une manière connue en soi.

**Revendications pour l'Etat Contractant: AT**

1. Un procédé de préparation de compositions pharmaceutiques qui consiste à mélanger de la 3 - diméthylamino - 7 - méthyl - 1,2 - (n - propylmalonyl) - 1,2 - dihydro - 1,2,4 - benzotriazine et au moins un composé choisi parmi la 3′,4′,7 - tris(β - hydroxyéthyl)rutine et le (+) - 2 - (3,4 - dihydroxyphényl) - 3,5,7 - chromanetriol et éventuellement un ou plusieurs antibiotiques et/ou un ou plusieurs éléments traces et/ou une ou plusieurs vitamines avec des excipients, supports, diluants utilisés de façon classique dans la préparation des compositions pharmaceutiques et à transformer le mélange obtenu en une composition pharmaceutique d′une manière connue en soi.

2. Un procédé selon la revendication 1, qui consiste à mélanger de 60 à 300 mg de 3 - diméthylamino - 7 - méthyl - 1,2 - (n - propylmalonyl) - 1,2 - dihydro - 1,2,4 - benzotriazine par unité de dosage.

3. Une procédé selon l'une quelconque des revendications 1 à 2, qui consiste à mélanger de 60 à 300 mg de 3',4',7 - tris(β - hydroxyéthyl)rutine et/ou (+) - 2 - (3,4 - dihydroxyphényl) - 3,5,7 - chromanetriol par unité de dosage.

4. Un procédé selon l'une quelconque des revendications 1 à 3, qui consiste à mélanger de la 3 - diméthylamino - 7 - méthyl - 1,2 - (n - propylmalonyl) - 1,2 - dihydro - 1,2,4 - benzotriazine et de la 3',4',7 - tris(β - hydroxyéthyl)rutine dans un rapport pondéral compris entre 2/1 à 1/1.

5. Un procédé selon l'une quelconque des revendications 1 à 4, qui consiste à mélanger de la 3 - diméthylamino - 7 - méthyl - 1,2 - (n - propylmalonyl) - 1,2 - dihydro - 1,2,4 - benzotriazine et du (+) - 2 - (3,4 - dihydroxyphényl) - 3,5,7 - chromanetriol dans un rapport pondéral compris entre 2/1 à 1/1.